(19) Europäisches Patentamt
European Patent Office
Office européen des brevets

(11) Publication number: **0 000 602**
**B1**

(12) **EUROPEAN PATENT SPECIFICATION**

(45) Date of publication of patent specification: **22.07.81**

(51) Int. Cl.³: **C 07 C 118/04**

(21) Application number: **78200097.0**

(22) Date of filing: **14.07.78**

(54) **Process for the preparation of isocyanates from formamides.**

(30) Priority: **02.08.77 NL 7708510**

(43) Date of publication of application:
**07.02.79 Bulletin 79/3**

(45) Publication of the grant of the European patent:
**22.07.81 Bulletin 81/29**

(84) Designated Contracting States:
**BE CH DE FR GB NL SE**

(56) References cited:
**FR - A - 2 291 186**
**FR - A - 2 310 334**
**GB - A - 1 152 978**
**US - A - 3 099 673**
**US - A - 3 277 140**
**US - A - 3 960 914**

(73) Proprietor: **Akzo N.V.**
**Ijssellaan 82**
**NL-6826 DW Arnhem (NL)**

(72) Inventor: **Heyboer, Nico**
**Balyeweg 2**
**NL-6874 AJ Wolfheze (NL)**

(74) Representative: **Sieders, René et al,**
**P.O. Box 314**
**NL-6800 AH Arnhem (NL)**

**EP 0 000 602 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the preparation of isocyanates from formamides.

The invention relates to a process for the preparation of isocyanates from formamides by contacting a formamide with a metal catalyst under the action of heat. A process of the type indicated above is known from, for instance, United States Patent Specification 3 960 914. According to the process described therein a formamide having the formula R—NHCHO, where R represents an organic group, is heated to a temperature in the range of 50° to 300°C in the presence of a dehydrogenation catalyst.

As drawbacks to technical application of this process may be mentioned the low yield, the low selectivity and the large amount of very costly catalyst which must be continuously regenerated.

The present invention provides a process which no longer shows these drawbacks.

The invention consists in that in a process of the known type indicated above N-monosubstituted formamides having the general formula

$$R(\overset{H}{\overset{|}{N}}-\overset{O}{\overset{\|}{C}}-H)_n,$$

where R represents a substituted or unsubstituted hydrocarbon radical and n is 1 or 2, are oxidized in gas phase with a molecular oxygen-containing gas fed into the reaction zone which is maintained at a temperature in the range of 300° to 600°C and in the presence, for a contacting time of 0.01 to 6 seconds, of a catalytic amount of copper and/or one or more metals of the groups IB and VIII of the 5th and 6th period of the Periodic System of Elements to yield the corresponding isocyanates having the general formula $R(NCO)_n$, where R and n have the above-mentioned meaning, and the resulting reaction mixture in gas phase is subjected to a separation treatment known per se, care being taken that any condensed isocyanate is immediately separated from the reaction mixture or dissolved into a water-immiscible, or practically water-immiscible, solvent.

It has been found that in this way at a very high degree of conversion (>95%) a yield can be obtained of more than 85% at a selectivity of more than 90%.

The results obtained by the process now proposed are surprising in that it was expected that the water set free in the reaction would at once combine with the isocyanate present and thus prevent isolation of the isocyanate. The applicant has found, however, that under the prevailing reaction conditions the affinity in the gas phase between isocyanates and water is so small that separation of these components in that phase is technically feasible.

In the process according to the invention use may be made of practically all N-monosubstituted formamide compounds which may be gasified under said reaction conditions and of which the substituents, if any, other than the N-monosubstituted formamide groups do not undergo any undesirable decomposition under the prevailing reaction conditions nor unduly poison the catalyst used.

R may represent a substituted or unsubstituted alkyl group, cycloalkyl group, aryl group, aralkyl group, or alkaryl group.

Group R will generally contain not more than 24 and preferably not more than 18 carbon atoms. As possible substituents may be mentioned chlorine, fluorine, cyano and alkyl carbonyl or alkoxy carbonyl preferably containing not more than 10 carbon atoms in the alkyl group or in the alkoxy group. The contacting time used will be very much dependent on other conditions, such as the type of catalyst, the reactivity of the formamide and the temperature.

It has been found that optimum results are usually obtained employing a contacting time in the range of 0.1 to 1.0 second.

Although satisfactory results may be obtained with copper and all further metals which are nobler than copper, it has been found preferable to make use of one or more of the following noble transition metals: Ru, Rh, Pd, Os, Ir, Pt, Au.

Optimum results were obtained using a silver catalyst.

The selectivity of this catalyst may, if desired, be further increased in the presence of a chloride, iodide or cyanide of Cu, Ru, Pd, Os, Ir or Pt in an amount which is 0.1 to 40 per cent by weight of the silver.

All the above-mentioned catalytically active metals may, if desired, form part of an alloy that may still contain other elements which may or may not be catalytically active in themselves.

Also the physical form of the catalyst may be of beneficial influence on the course of the reaction.

Thus, the use of silver catalyst gave satisfactory results when the silver was present in the form of crystals.

Satisfactory results were also obtained when as carrier material carborundum was used. Optimum results were obtained when the silver catalyst was present in the form of silver wool.

The temperature at which the reaction can be satisfactorily carried out is in the range of 300° to 600°C.

At a temperature below 300°C too little formamide is converted, whereas at a temperature above 600°C sidereactions may cause the yield to drop so much that economically the process is no longer attractive.

It has been found that for most N-monosubstituted formamide compounds the reaction proceeds optimally at a temperature in the

range of 350° to 450°C. The course of the reaction is, naturally, also influenced by other process conditions, such as pressure, type of catalyst, type of reactor, residence time, velocity of the gas, the ratio of formamide to oxygen, and the concentration of the formamide in the gas stream.

The proportion of formamide in the reaction mixture at the start of the reaction will generally be chosen between 0.1 and 10 per cent by volume.

Favourable results will generally be obtained in the presence per formamide group of an at least an equivalent amount of oxygen.

The volume percentage of oxygen in the reaction mixture at the start of the reaction will generally be in the range of 0.05 to 10 per cent by volume. A higher percentage may not only have a detrimental effect on the yield, but it also carries with it the risk of flammability of the reaction mixture.

Satisfactory results will be obtained when the percentage by volume of oxygen in the reaction mixture at the start of the reaction is chosen between 0.5 and 5 per cent by volume. The reaction will generally be carried out in the presence of a high excess of inert gas. Consequently, the partial pressure of the gases taking part in the reaction will only be a fraction of the absolute pressure of the gas mixture. The latter pressure may vary from <100 kPa to 1000 kPa or higher. In order to facilitate controlling the feed stock of formamide and/or other additives the reaction mixture may contain inert solvent in addition to inert gas. As examples of these solvents may be mentioned hydrocarbons such as benzene, toluene, ethyl benzene, xylene, biphenyl, n-pentane, n-hexane, n-heptane, cyclopentane, cyclohexane, methylcyclopentane, nitriles such as benzonitrile, tolunitrile and adipodinitrile; esters such as the octyl esters of acetic acid and butyric acid; 1-methyl-naphthalene and tetrahydronaphthalene. Surprisingly, it has been found that the selectivity of the reaction may still be considerably improved if it is carried out in the presence of a chlorinated organic compound. As examples thereof may be mentioned methyl chloride, ethyl chloride, dichloroethane, chlorinated polyphenyl compounds, chlorinated biphenyl, o-dichlorobenzene or mixtures of these compounds. The selectivity of the reaction can also be improved in the presence of sulphur, hydrogen sulphide and/or an organic sulphur compound the sulphur of which is in divalent form. As an example of an organic compound also carbon disulphide can be mentioned. Other examples are thioalcohols such as methane thiol, butane thiol, thio-ethers, thioacetals, thiol esters, thiophene and homologous compounds. The amounts to be used thereof in the reaction mixture may vary from a few p.p.m. by volume up to as much as more than the equivalent amount by weight of the N-monosubstituted formamide compounds.

It has been found that very good results may be obtained if the amount of the chlorinated organic compound and/or the amount of S, $H_2S$ and/or organic sulphur compound in the reaction mixture is chosen between 1 and 100 p.p.m. by volume.

Upon conclusion of the reaction rapid steps should be taken to prevent water and isocyanate from entering into reaction with each other.

This problem may be solved in various ways.

One solution consists in that upon termination of the reaction the reaction mixture is rapidly cooled, after which the $H_2O$-containing phase and the isocyanate-containing phase are separated from each other as fast as possible by a separation method known in itself, for instance a physical separation method such as filtration and/or extraction. As a disadvantage to this solution it may be put forward that the cost of rapid cooling may run up very high. Moreover, there is always the risk of the isocyanate reacting with water in the case of insufficient or insufficiently rapid cooling.

Another solution provided by the invention consists in that upon termination of the reaction, but prior to condensation of the isocyanate, the reaction mixture is passed over a water-absorbing agent. As examples of water-absorbing agents may be mentioned magnesium sulphate, sodium sulphate and/or calcium chloride.

As the water must be eliminated prior to or during condensation of the isocyanate, it is preferred that use should be made of a drying agent which is still satisfactorily effective at relatively high temperatures.

It has been found that this last-mentioned requirement can very well be satisfied by using a molecular sieve, preferably of the A3 type.

Surprisingly, it has been found that rapid cooling or drying of the reaction mixture prior to condensation of the isocyanate is no longer required, provided that care is taken that condensation of the isocyanate is effected in the presence of a water-immiscible or practically water-immiscible solvent for the isocyanate.

As examples of suitable solvents may be mentioned benzene, toluene, xylene, chlorinated hydrocarbons such as carbon tetrachloride, trichloroethylene, ethylene dichloride and various isomers of chlorobenzene, such as 1,3-dichlorobenzene. Depending on the temperature to which the solvent-containing reaction mixture is cooled the water formed in the reaction will condense or not. If use is made of sufficient solvent and a not unduly long contacting time, the percentage isocyanate which will decompose as a result of its reacting with water of condensation, if any, is practically negligible. If these two requirements are difficult to satisfy, if at all, then the invention provides a process which is so carried out that a water-absorbing agent is present during or after

condensation of the isocyanate.

To this end the same water-absorbing agents may be employed as mentioned above.

An alternative process in which rapid cooling or drying of the reaction mixture prior to condensation of the isocyanate is no longer required consists in that the reaction mixture emerging from the reaction zone, after having been cooled to some degree or not, is passed into a water-immiscible or practically water-immiscible solvent for the isocyanate. Upon condensation, if any, of the water formed during the reaction, it may be separated in the form of an immiscible phase. Optionally, a finely divided water-absorbing agent may be suspended in the solvent. Both for the solvent and the water-absorbing agent the same substances may be used as indicated above.

The solvent present during condensation of the isocyanate may be incorporated into the reaction mixture during, before or after the reaction. It may be added in the liquid or in the gaseous state.

The amount of solvent should be so chosen that it is capable of absorbing as much as possible of the isocyanate formed.

An attractive method of adding the solvent consists in the solvent being sprayed into the reaction mixture emerging from reaction zone.

In order as much as possible to avoid the use of a water separator and/or drying agents the temperature at which the solvent-isocyanate mixture is caused to condense or the temperature of the solvent through which the reaction mixture is passed should be so chosen that it is just above the dew point of the water contained in the reaction mixture after both the solvent and the isocyanate have been separated therefrom. Especially in order to avoid that the isocyanate condenses at an earlier moment than the solvent for the isocyanate such a solvent for the isocyanate should be chosen that its boiling point is not lower than 150°C. It is preferred that use be made of a solvent having a boiling point in the range of about 200° to about 300°C. As examples of suitable solvents may be mentioned aromatic hydrocarbons such as cumene, pseudo cumene, biphenyl, $\alpha$-methyl naphthalene; aliphatic and cycloaliphatic hydrocarbons such as decane, hexahydrocumene, aromatic halohydrocarbons such as orthodichlorobenzene, bromobenzene, $\alpha$-chloronaphthalene, esters such as the octyl esters of acetic acid and butyric acid; nitriles such as adipodinitrile, benzonitrile, and ketones such as benzophenone.

The oxidation reaction in the gas phase of the N-monosubstituted formamides to isocyanates can be conducted in a continuous or batch operation.

It is preferred, however, that the reaction should be carried out continuously. Separation of the resulting reaction mixture may again be carried out continuously or batchwise. Here too, preference is given generally to a continuous process.

As examples of organic isocyanates that may be prepared by the process according to the invention may be mentioned hexyl isocyanate, octyl isocyanate, dodecyl isocyanate, octadecyl isocyanate, tetramethylene diisocyanate, pentamethylene diisocyanate, hexamethylene diisocyanate, octamethylene diisocyanate, undecamethylene diisocyanate, dodecamethylene diisocyanate, cyclohexyl isocyanate, $\beta$-naphthyl isocyanate, xylene diisocyanate, diphenyl methane-4,4′-diisocyanate, benzyl isocyanate, phenylethyl isocyanate, phenyl isocyanate, methyl isocyanate, ethyl isocyanate, propyl isocyanate, eicosyl isocyanate, tetracosyl isocyanate, p-dodecylphenyl isocyanate, 3-chloro-4-octylphenyl isocyanate, 2,4-toluene diisocyanate, 2,6-toluene diisocyanate, and mixtures of the afore-mentioned compounds. The following examples are given to illustrate the process of the present invention. They are, of course, not to be regarded as limiting the scope thereof.

## Examples I—IX

In these examples use is made of a reactor which is schematically illustrated in Figure 1. In it the numeral 1 refers to a stainless steel tubular reactor 70 cm long and 2.5 cm in diameter.

The tubular reactor is provided with a heating jacket 2 accommodating electric heating elements. The temperature of the reactor is kept at a pre-set value by means of two thermocouples. The tube 3 running along the centre line of the tubular reactor contains thermocouples 4, 5, 6, 7, 8 and 9, which are connected to a recorder 10. The hatched part of the reactor is filled with carborundum and the cross-hatched part contains the catalyst. Near the thermocouple 8 ends a stainless steel tube 11 through which a high-boiling solvent 13 was passed which, upon evaporation, immediately mixed with the reaction mixture emerging from the catalyst bed. The mixture 14 consisting of the solvent 13 and the components of the reaction mixture was passed to a cooler. The feed stock of the reactor was formed by a gaseous mixture 12, which had been obtained by dropwise charging a liquid mixture of formamide and a liquid which is chemically inert to the reactants, to a carborundum-filled vertically positioned glass evaporator through which there was passed a nitrogen stream into which a particular amount of air or oxygen had been taken up. The evaporator was heated in an oil bath to a temperature in the range of about 160° to 200°C.

The condensor consisted of a glass tube provided with a water jacket and was filled with small glass rings. The condensor was set to the mixture 14 being cooled down to 25°C. Under these conditions there was no or hardly any

condensation of water. The composition of the condensed mixture was analysed gaschromatographically with a 1 m glass OV 225 column at a temperature of 100° to 250°C.

For a rapid gaschromatographic determination a liquid inert to the reactant was fed into the reactor as internal reference along with the formamide. In each run the feed stock 12 was obtained by evaporation at a rate of 5 ml/hour of a mixture of equal amounts of weight of phenyl formamide and benzonitrile (internal reference for the gaschromatographic analysis) in a gas stream consisting of nitrogen (48—120 1/hour) and air (3.5—16 1/hour). As high-boiling solvent 13 for the isocyanate 1-chloronaphthalene or 1-bromonaphthalene was used (30 ml/hour). The results are given in the following table.

TABLE

| Example | Catalyst | Temp. catalyst bed (°C) | Volume percentages in feed stock | | Residence time in catalyst bed (seconds) | Phenyl isocyanate yield (%) | Selectivity (%) |
|---|---|---|---|---|---|---|---|
| | | | oxygen | phenyl formamide | | | |
| I | 5% Pt on quartz wool | 380 | 2.5 | 0.9 | 2.0 | 9 | 18 |
| II | Copper tape | 390 | 2.5 | 0.8 | 1.5 | 16 | 35 |
| III | Pd on glass wool | 345 | 1.0 | 0.4 | 0.4 | 6 | 9 |
| IV | Pd on glass wool | 350 | 4.1 | 0.7 | 0.5 | 8 | 14 |
| V | Ru on glass wool | 310 | 3.0 | 0.7 | 0.5 | 2 | 19 |
| VI | Ir on glass wool | 400 | 1.5 | 0.4 | 0.2 | 3 | 6 |
| VII | Ag–wool | 350 | 0.75 | 0.55 | 0.5 | 59 | 66 |
| VIII | Ag–crystals | 375 | 1.2 | 0.75 | 0.5 | 48 | 58 |
| IX | Ag on carborundum | 345 | 0.8 | 0.4 | 0.2 | 26 | 38 |

Example X

The procedure followed in this example was entirely in accordance with that used in the preceding examples, except that the feed stock consisted of a mixture of equal amounts by weight of n-hexyl formamide and benzonitrile. This mixture was evaporated at a rate of 5 ml/hour in a gas stream consisting of nitrogen (98 litres/hour) and air (1.2 litres/hour). The catalyst bed had a volume of 30 ml and contained 20 grammes of silver wool the filaments of which had a diameter of 0.03 mm. The temperature was 410°C. The mean residence time in the catalyst bed was 0.5 seconds. As high-boiling solvent 1-bromonaphthalene was fed to the reactor at a rate of 30 ml/hour. After a few hours the yield of n-hexyl isocyanate stabilized to 81% and the selectivity to 85%, calculated on the amount of formamide used.

Example XI

The procedure in this example was entirely in accordance with that used in Example X, except that the starting mixture was evaporated at a rate of 30 ml/hour in a gas stream of nitrogen (117 litres/hour) and air (7.2 litres/hour). As high-boiling solvent 1-bromonaphthalene was supplied at a rate of 100 ml/hour. The mean residence time was 0.35 seconds. The yield of n-hexyl isocyanate was 85% and the selectivity 91%.

Example XII

In accordance with the procedure described in Example XI n-hexyl formamide was evaporated at a rate of 10.5 g/hour in a gas stream of nitrogen (120 litres/hour) and air (5.5 litres/hour). As high boiling solvent 1-methyl naphthalene was used. The n-hexyl isocyanate yield was determined both gaschromatographically (68%) and by titration with dibutylamine in accordance with the method of David and Staley in High Polymers Vol. XVI Analytical Chem. of Polyurethanes Part III (1969) 87 (Wiley Interscience). This method gave a yield of 70%. The selectivity was 82%.

Example XIII

This example was carried out entirely in accordance with Example X, except that the feed stock was evaporated at a rate of 6 ml/hour. Nitrogen was fed at a rate of 100 litres/hour and air at a rate of 1.7 litres/hour. To the gas stream there was moreover fed 0.25% of 1,2-dichloroethane, calculated on the amount by weight of hexyl formamide. The mean residence time weas 0.45 seconds. As high-boiling solvent 1-chloronaphthalene was charged to the reactor at a rate of 30 ml/hour. The use of a reaction temperature of 420°C and a catalyst bed of 20 ml (20g) of silver wool gave n-hexyl isocyanate in 67% yield at a selectivity of 97%.

Example XIV

Use being made of the procedure of Example X a feed mixture of equal amounts by weight of n-hexyl formamide and benzonitrile was evaporated at a rate of 9 ml/hour in a gas stream of nitrogen (48 litres/hour) and air (2 litres/hour). This gas mixture was passed over an 8 cm-long catalyst bed of 15 grammes of silver wool at a temperature of 425°C. The residence time was 0.44 seconds. The feed rate of 1-chloronaphthalene was 50 ml/hour. The condensor was set to the reaction mixture being cooled down to a temperature of about 0°C, which resulted in the separation in the condensor of two immiscible liquid phases.

The upper phase consisted of water, the other of 1-chloronaphthalene in which n-hexyl isocyanate and non-converted n-hexyl formamide were dissolved. After separation of the two phases the amount of n-hexyl isocyanate was determined. The yield calculated from it was 76% at a selectivity of 94%.

Example XV

This example was carried out entirely in accordance with the procedure described in Example XIV, except that the water was not separated but bound with the aid of drying agents.

In independent experiments with anhydrous sodium sulphate, magnesium sulphate or molecular sieve A3, respectively, the results obtained were similar to those obtained in Example XIV.

Example XVI

This example was carried out entirely in accordance with the procedure given in Example XIV, except that the small glass rings in the condenser had been replaced with molecular sieve A3. It was found that both the yield and the selectivity were the same as obtained in Example XIV.

Example XVII

Per hour 9 ml of a mixture of equal amounts by weight of n-hexyl formamide and benzonitrile were evaporated in a gas stream (91 litres of $N_2$ and 2 litres of air). The procedure used was entirely in accordance with that of Example XIV, except that the catalyst consisted of 5 ml (about 5 grammes) of silver wool. At a temperature of 590°C this resulted in a residence time of 0.065 seconds. As high-boiling solvent there was added 1-chloronaphthalene at a rate of 48 ml/hour. The yield of n-hexyl isocyanate was 12% at a selectivity of 28%.

Example XVIII

The experiment of Example XVII was repeated in such a way that a catalyst bed of only 2 ml was used. The $N_2$ was fed at a rate of 106 litres per hour along with air at a rate of 2.2 litres per hour. The residence time was 0.025

seconds at 495°C. The yield of isocyanate was 32% at a selectivity of 57%.

Example XIX

Per hour 5.16 grammes of cyclohexyl formamide was evaporated in a gas stream (100 litres of $N_2$ and 5 litres of air). The starting mixture was passed over 20 ml of silver wool at a temperature of 440°C, which corresponded to a mean residence time of 0.4 seconds. The procedure was further entirely in accordance with that of Example X, except that as high-boiling solvent 1-chloronaphthalene was charged to the reactor at a rate of 50 ml/hour. The yield of cyclohexyl isocyanate was determined gaschromatographically and found to be 76% at a selectivity of 79%. Titration with dibutylamine in accordance with the method described in the publication of David and Staley mentioned in Example XII also showed a yield of 76%.

Example XX

The experiment was carried out entirely in accordance with the procedure given in Example XIX, except that a mixture of equal amounts by weight of n-hexyl formamide, benzonitrile and o-dichlorobenzene was evaporated at a rate of 9 ml/hour in a gas stream consisting of nitrogen (90 litres/hour) and air (9 ml/hour). The gas mixture was passed over 20 ml of silver wool at a temperature of 410°C, which corresponded to a mean residence time of 0.5 seconds. As high-boiling solvent 1-chloronaphthalene was charged to the reactor at a rate of 30 ml/hour. The yield of n-hexyl isocyanate was 41% at a selectivity of 72%. The o-dichlorobenzene could be recovered quantitatively.

Example XXI

This example is entirely in accordance with Example XIX, except that use was made of a mixture of equal amounts by weight of benzyl formamide and 1-methyl naphthalene which was evaporated at a rate of 5.7 grammes per hour in a gas stream of nitrogen (118 litres/hour) and air (1.4 litres/hour). The temperature of the catalyst bed was 415°C, the mean residence time 0.4 seconds. As high-boiling solvent 1-chloronaphthalene was charged to the reactor at a rate of 30 ml/hour. The yield of benzyl isocyanate was 57% at a selectivity of 80%.

Example XXII

The experiment of Example X was repeated in such a way that a mixture of phenyl formamide and benzonitrile was evaporated at a rate of 5 ml/hour in a gas stream of 110 litres of nitrogen and 3 litres of air/hour. The gas mixture also comprised carbon disulphide, which was fed at a rate of 0.67% per hour, calculated on the added amount by weight of n-hexyl formamide. Use of the same catalyst bed as in Example XIX at a temperature of 375°C resulted in a yield of phenyl isocyanate of 41% at a selectivity of 74%.

Example XXIII

The experiment of Example XIX was repeated in such a way that a mixture of equal parts by weight of m-tolyl formamide and benzonitrile was evaporated at a rate of 5 ml/hour in a gas stream of nitrogen and air fed at rates of 118 litres and 2.8 litres, per hour, respectively. 1-chloronaphthalene was charged to the reactor at a rate of 30 ml/hour. At a temperature of 415°C the yield of m-tolyl isocyanate was 70% and the conversion of the formamide was practically quantitative.

Example XXIV

In this example as well as in the following ones use was made of a somewhat varied set up of the type of reactor employed in the preceding examples. The apparatus is schematically illustrated in Figure 2. In it the numeral 1 refers to a stainless steel tubular reactor 70 cm long and 2 cm in diameter. The vertically positioned tubular reactor was provided with a stainless steel jacket 2 accommodating electric heating elements. Above the jacket 2 the tubular reactor ends in a space 3 to which there is connected a line 4 for the supply of the gas stream. Along the centre line of the space 3 there is provided a stainless steel tube 5 which ends in a capillary tube 6. The feed stock of mono- and/or diformamide along with, if desired, some solvent is charged to the reactor through the stainless steel tube 5. The distance from the lower end of the capillary tube to the upper end of the hatched part 7 inthe tubular reactor is about 20 cm. The hatched part 7 comprises a carborundum bed 15 cm long. Catalyst bed 8, which in the experiments described hereinafter exclusively consisted of silver wool, is the cross hatched part below the carborundum bed 7, and the hatched part 9 below the bed 8 is a carborundum bed 9—13 cm long. Into this bed ends a stainless steel tube 10 through which in all experiments a high-boiling solvent was passed.

Under the reactor 1 is a glass condensor 11 which is filled with glass beads. The low-volatile components are collected in a sampling bottle 12 and subsequently analysed gaschromatographically with a 1 m glass OV 225 column. In the following examples the mixture fed through the tube 5 had already been heated to a temperature of about 100°C. The temperature had further been so set that at the end of the capillary tube 6 it was about 150°C. At the top of the carborundum bed 7 the temperature was about 300°C and gradually increased to the temperature of the catalyst bed 8 (about 410° to 470°C).

In the carborundum bed 9 the temperature finally decreased to about 300°C. Evaporation of all the liquids was realized with the aid of a

gas stream through the line 4, the feed rate of which varied from 108 to 136 litres per hour for nitrogen and from 0.9 to 10 litres per hour for air.

The temperature of the condenser was so set that the reaction mixture left the condenser at a temperature of approximately 25°C.

Through the tube 5 there was fed at a rate of 9 ml/hour a mixture of equal amounts by weight of n-octadecyl formamide and m-tolunitrile (internal reference for the gaschromatographical determination). This mixture was evaporated in a gas stream of nitrogen (110 litres/hour) and air (3.0 litres/hour). The amount of catalyst (silver wool) was 20 ml (20.0 grammes). The temperature was 470°C. As high-boiling solvent 1-chloronaphthalene was fed through the tube 10 at a rate of 30 ml/hour.

The yield of octadecyl isocyanate was 47%. At a conversion of 72% this corresponded to a selectivity of 65%.

### Example XXV

The procedure in this example was entirely in accordance with that of the preceding example, except that the starting material consisted of a mixture of equal amounts by weight of m-methoxy carbonylphenyl formamide and m-tolunitrile fed to the reactor at a rate of 4.5 ml/hour. This mixture was evaporated in a gas stream of nitrogen (136 litres/hour) and air (0.9 litres/hour). The temperature of the catalyst bed (15 ml) was 410°C. The residence time was 0.17 seconds. As high-boiling solvent for the isocyanate tetralin was added at a rate of 30 ml/hour.

The yield of m-methoxycarbonylphenyl isocyanate was 53% at a selectivity of 62%.

### Example XXVI

The experiment of Example XXV was repeated in such a way that a mixture of m-cyanophenyl formamide, m-tolunitrile and benzonitrile in a weight ratio of 1:1:5 was fed to the reactor at a rate of 12 ml/hour. The mixture was evaporated in a gas stream of nitrogen (108 litres/hour) and air (1.8 litres/hour).

The yield of m-cyanophenyl isocyanate was 29% at a virtually quantitative conversion.

### Example XXVII

In accordance with the procedure used in Example XXV a mixture of hexamethylene diformamide, adiponitrile (internal reference for the gaschromatographical determination) and biophenyl in a weight ratio of 1:1:5 was fed at a rate of 12 ml/hour. The gas stream consisted of nitrogen (110 litres/hour) and air (10 litres/hour). The residence time in the catalyst bed (20 ml, or about 20 grammes of silver wool, the filaments of which had a diameter of 0.03 mm) was 0.25 seconds at a temperature of 430°C. As high-boiling solvent 1-chloronaphthalene was added at a rate of 30 ml/hour. The conversion was found to be quantitative. The yield of hexamethylene diisocyanate was 30%.

### Example XXVIII

The experiment of Example XXVII was repeated in such a way that use was made of 12 ml/hour of a mixture of decamethylene diformamide adiponitrile (internal reference) and biphenyl in a weight ratio of 1:1:5. The conversion was again quantitative, with decamethylene diisocyanate being obtained in 31% yield.

### Example XXIX

In the same way as indicated in Example XXVII 12 ml/hour of a mixture of 2,4-toluene diformamide, benzonitrile (internal reference) and gamma-butyrolactone in a weight ratio of 1:1:5 were evaporated in a gas stream of 120 l/hour $N_2$ and 8.5 l/hour air. The residence time in the catalyst bed was 0.2 seconds at a temperature of 430°C. The conversion was quantitative and the yield of 2,4-toluene diisocyanate 27%.

### Example XXX

In the same way as indicated in Example XXVII 12 ml/hour of a mixture of m-xylylene diformamide, 1-methyl-naphthalene (internal reference) and gamma-butyrolactone in a weight ratio of 1:1:5 were evaporated in a gas stream of 112 l/hour $N_2$ and 6.0 l/hour air.

The conversion was quantitative and the yield of m-xylylene diisocyanate 21%.

## Claims

1. A process for the preparation of isocyanates by contacting a formamide with a metal catalyst under the action of heat, characterized in that N-monosubstituted formamides having the general formula

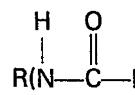

where R represents a substituted or unsubstituted hydrocarbon radical and n is 1 or 2, are oxidized in gas phase with a molecular oxygen-containing gas fed into the reaction zone which is maintained at a temperature in the range of 300° to 600°C and in the presence, for a contacting time of 0.01 to 6 seconds, of a catalytic amount of copper and/or one or more metals of the groups IB and VIII of the 5th and 6th period of the Periodic System of Elements to yield the corresponding isocyanates having the general formula $R(NCO)_n$, where R and n have the above-mentioned meaning, and the resulting reacting mixture in gas phase is subjected to a separation treatment per se, care being taken that any condensed isocyanate is immediately separated from the reaction mixture or dissolved into a water-immiscible, or practically water-immiscible, solvent.

2. A process according to claim 1, characterized in that the contacting time is in the range of 0.1 to 1 second.

3. A process according to claim 1 or 2, characterized in that the reaction is carried out in the presence of a silver catalyst.

4. A process according to claim 3, characterized in that the silver catalyst is present in the form of silver wool.

5. A process according to one or more of the preceding claims, characterized in that the reaction is carried out at a temperature in the range of 350° to 450°C.

6. A process according to one or more of the preceding claims, characterized in that at the start of the reaction the proportion of formamide in the reaction mixture is in the range of 0.1 to 10 per cent by volume.

7. A process according to one or more of the preceding claims, characterized in that per formamide group at least an equivalent amount of oxygen is present.

8. A process according to one or more of the preceding claims, characterized in that at the start of the reaction the proportion of oxygen in the reaction mixture is in the range of 0.05 to 10 per cent by volume.

9. A process according to claim 8, characterized in that at the start of the reaction the proportion of oxygen in the reaction mixture is in the range of 0.5 to 5 per cent by volume.

10. A process according to one or more of the preceding claims, characterized in that the reaction is carried out in the presence of a chlorinated organic compound.

11. A process according to one or more of the claims 1—9, characterized in that the reaction is carried out in the presence of S, $H_2S$ and/or an organic sulphur compound in which the sulphur is in divalent form.

12. A process according to one or more of the preceding claims, characterized in that upon conclusion of the reaction the reaction mixture is rapidly cooled, after which the $H_2O$-containing phase and the isocyanate-containing phase are separated from each other as fast as possible by a separation method known per se.

13. A process according to one or more of the preceding claims, characterized in that upon conclusion of the reaction but prior to condensation of the isocyanate the reaction mixture is passed over a water-absorbing agent.

14. A process according to one or more of the claims 1—11, characterized in that condensation of the isocyanate is effected in the presence of a water-immiscible or practically water-immiscible solvent for the isocyanate.

15. A process according to claim 14, characterized in that during or after condensation of the isocyanate a water-absorbing agent is present.

16. A process according to one or more of the claims 1—11, characterized in that the reaction mixture emerging from the reaction zone after it has been cooled to some degree or not is passed into a water-immiscible or practically water-immiscible solvent for the isocyanate.

17. A process according to claim 16, characterized in that in the solvent there is suspended a finely divided water absorbing agent.

18. A process according to claim 14, characterized in that the temperature at which the solvent-isocyanate mixture is condensed is so chosen that it is just above the dew point of the water present in the reaction mixture after the separation therefrom of both the solvent and the isocyanate.

19. A process according to claim 16, characterized in that the temperature of the solvent through which the reaction mixture is passed is so chosen that it is just above the dew point of the water contained in the reaction mixture after separation therefrom of both the solvent and the isocyanate.

## Patentansprüche

1. Verfahren zur Herstellung von Isocyanaten, in dem man ein Formamid bei erhöhter Temperatur mit einem Metallkatalysator in Kontakt bringt, dadurch gekennzeichnet, dass N-monosubstituierte Formamide mit der allgemeinen Formel

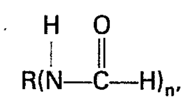

wobei R ein substituiertes oder nicht substituiertes Kohlenwasserstoff radikal darstellt und n=1 oder 2, in der gasförmigen Phase mit einem molekularen Sauerstoff enthaltendem Gas, und zwar in der Reaktionszone bei einer Temperatur von 300° bis 600°C und während einer Kontakzeit von 0,01 bis 6 Sekunden in Anwesenheit einer katalytischen Menge Kupfer und/oder eines oder mehr Metalle der Gruppen IB und VIII der 5. und 6. Periode des Periodensystems der Elemente, oxydiert werden zu den entsprechenden Isocyanaten mit der allgemeinen Formel $R(NCO)_n$, wobei R und n die obengenannte Bedeutung haben, und das erhaltene Reaktionsgemisch in der gasförmigen Phase einer für sich bekannten Abscheidungsbehandlung unterzogen wird, wobei dafür Sorge zu tragen ist, dass eventuell kondensiertes Isocyanat sofort vom Reaktionsgemisch abgeschieden oder in einem nicht oder praktisch nicht mit Wasser mischbaren Lösungsmittel aufgelöst wird.

2. Verfahren gemäss Anspruch 1, dadurch gekennzeichnet, dass die Kontaktzeit im Bereich 0,1 bis 1 Sekunde liegt.

3. Verfahren gemäss Anspruch 1 oder 2, dadurch gekennziechnet, dass die Reaktion in

Anwesenheit eines Silberkatalysators erfolgt.

4. Verfahren gemäss Anspruch 3, dadurch gekennzeichnet, dass der Silberkatalysator in Form von Silberwolle vorhanden ist.

5. Verfahren gemäss einem oder mehreren der vorangehenden Ansprüche, dadurch gekennzeichnet, dass die Reaktion bei einer Temperatur zwischen 350° und 450°C durchgeführt wird.

6. Verfahren gemäss einem oder mehreren der vorangehenden Ansprüche, dadurch gekennzeichnet, dass der Anteil des Formamids im Reaktionsgemisch zu Beginn der Reaktion zwischen 0,1 und 10 Volumenprozent liegt.

7. Verfahren gemäss einem oder mehreren der vorangehenden Ansprüche, dadurch gekennzeichnet, dass je Formamid-Gruppe zuminddest eine äquivalente Sauerstoffmenge vorhanden ist.

8. Verfahren gemäss einem oder mehreren der vorangehenden Ansprüche, dadurch gekennzeichnet, dass der Sauerstoffanteil im Reaktionsgemisch zu Beginn der Reaktion zwischen 0,5 und 10 Volumenprozent liegt.

9. Verfahren gemäss Anspruch 8, dadurch gekennzeichnet, dass der Sauerstoffanteil im Reaktionsgemisch zu Beginn der Reaktion zwischen 0,5 und 5 Volumenprozent liegt.

10. Verfahren gemäss einem oder mehreren der vorangehenden Ansprüche, dadurch gekennzeichnet, dass die Reaktion in Anwesenheit einer chlorierten organischen Verbindung durchegeführt wird.

11. Verfahren gemäss einem oder mehreren der Ansprüche 1—9, dadurch gekennzeichnet, dass die Reaktion in Anwesenheit von S, $H_2S$ und/oder einer organischen Schwefelverbindung durchgeführt wird, in der der Schwefel sich in zweiwertiger Form befindet.

12. Verfahren gemäss einem oder mehreren der vorangehenden Ansprüche, dadurch gekennzeichnet, dass das Reaktionsgemisch nach erfolgter Reaktion schnell abgekühlt wird, wonach die $H_2O$ enthaltende Phase und die Isocyanat enthaltende Phase so schnell wie möglich nach einer für sich bekannten Methode voneinander getrennt werden.

13. Verfahren gemäss einem oder mehreren der vorangehenden Ansprüche, dadurch gekennzeichnet, dass das Reaktionsgemisch nach erfolgter Reaktion, jedoch vor der Kondensation des Isocyanats, über einen wasserabsorbierendes Mittel geleitet wird.

14. Verfahren gemäss einem oder mehreren der Ansprüche 1—11, dadurch gekennzeichnet, dass die Kondensation des Isocyanats in Anwesenheit eines nicht oder praktisch nicht mit Wasser mischbaren Lösungsmittels für das Isocyanat erfolgt.

15. Verfahren gemäss Anspruch 14, dadurch gekennzeichnet, dass während oder nach der Kondensation des Isocyanats ein wasserabsorbierendes Mittel vorhanden ist.

16. Verfahren gemäss einem oder mehreren der Ansprüche 1—11, dadurch gekennzeichnet, dass das aus der Reaktionszone tretende Reaktionsgemisch nach dessen teilweisen Abkühlung oder nicht in ein nicht oder praktisch nicht mit Wasser mischbares Lösungsmittel für das Isocyanat geleitet wird.

17. Verfahren gemäss Anspruch 16, dadurch gekennzeichnet, dass im Lösungsmittel ein wasserabsorbierendes Mittel in feiner Verteilung suspendiert ist.

18. Verfahren gemäss Anspruch 14, dadurch gekennzeichnet, dass die Temperatur, bei der das Lösungsmittel/Isocyanatgemisch kondensiert wird, derart gewählt wird, dass sie gerade über dem Kondensationspunkt des im Reaktionsgemisch vorhandenen Wassers liegt, nachdem sowohl das Lösungsmittel als auch das Isocyanat davon abgeschieden worden sind.

19. Verfahren gemäss Anspruch 16, dadurch gekennzeichnet, dass die Temperatur des Lösungsmittels, durch das das Reaktionsgemisch geleitet wird, so gewählt wird, dass sie gerade über dem Kondensationspunkt des im Reaktionsgemisch enthaltenen Wassers liegt, nachdem sowohl das Lösungsmittel als auch das Isocyanat davon abgeschieden worden sind.

**Revendications**

1. Procédé pour préparer des isocyanates en mettant un formamide en contact avec un catalyseur métallique sous l'action de chaleur, caractérisé en ce que des formamides N-monosubstitués dela formule générale

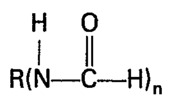

où R représente un radical hydrocarbure substitué ou non substitué et n=1 ou 2, sont oxydés en phase gazeuse avec un gas contenant de l'oxygène moléculaire, amené à la zone de réaction, qui est maintenue à une température située dans la gamme de 300 à 600°C et en présence, pour une durée de contact de 0,01 à 6 secondes, d'une quantité catalytique de cuivre et/ou d'un ou plusieurs métaux des groupes IB VIII des 5[e] et 6[e] périodes du Tableau Périodique des Eléments pour obtenir les isocyanates correspondants ayant la formule générale $R(NCO)_n$, où R et n ont les significations susmentionnées, et en ce que le mélange réactionnel obtenu est soumis en phase gazeuse à un traitement de séparation connu en lui-même, en ayant soin que de l'isocyanate éventuellement condensé soit séparé immédiatement du mélange réactionnel ou dissous dans un solvant immiscible ou pratiquement immiscible à l'eau.

2. Procédé selon la revendication 1, caractérisé en ce que la durée de contact se situe dans la gamme de 0,1 à 1 seconde.

3. Procédé selon la revendication 1 ou 2, caractérisé en ce que la réaction est effectuee en présence d'un catalyseur d'argent.

4. Procédé selon la revendication 3, caractérisé en ce que le catalyseur d'argent est sous forme de laine d'argent.

5. Procédé selon l'une quelconque des revendications 1—4, caractérisé en ce que la réaction est effectuée à une température située dans la gamme de 350 à 450°C.

6. Procédé selon l'une quelconque des revendications 1—5, caractérisé en ce que, au début de la réaction, la quantité de formamide dans le mélange réactionnel se situe dans la gamme de 0,1 à 10% en volume.

7. Procédé selon l'une quelconque des revendications 1—6, caractérisé en ce que, par groupe formamide, une quantité au moins équivalente d'oxygène est présente.

8. Procédé selon l'une quelconque des revendications 1—7, caractérisé en ce que, au début de la réaction, la quantité d'oxygène dans le mélange réactionnel se situe dans la gamme de 0,05 à 10% en volume.

9. Procéde selon la revendication 8, caractérisé en ce que, a début de la réaction, la quantité d'oxygène dans le mélange reactionnel se situe dans la gamme de 0,5 à 5% en volume.

10. Procéde selon l'une quelconque des revendications 1—9, caractérisé en ce que la réaction est effectuée en présence d'un composé organique chloré.

11. Procédé selon l'une quelconque des revendications 1—9, caractérisé en ce que la réaction est effectuée en présence de S, de $H_2S$ et/ou d'un composé organique de soufre, dans lequel le soufre est sous forme bivalente.

12. Procédé selon l'une quelconque des revendications 1—11, caractérisé en ce que, au bout de la réaction, le mélange réactionnel est refroidi rapidement, après quoi la phase contenant le $H_2O$ et celle contenant l'isocyanate sont séparées l'une de l'autre le plus vite possible suivant un procédé connu en lui-même.

13. Procédé selon l'une quelconque des revendications 1—12, caractérisé en ce que, au bout de la réaction, mais avant la condensation de l'isocyanate, le mélange réactionnel est conduit sur un agent absorbant l'eau.

14. Procédé selon l'une quelconque des revendications 1—11, caractérisé en ce que la condensation d'isocyanate est effectuée en présence d'un solvant immiscible ou pratiquement immiscible à l'eau pour l'isocyanate.

15. Procédé selon la revendication 14, caractérisé en ce que, pendant ou après la condensation, un agent absorbant l'eau est présent.

16. Procédé selon l'une quelconque des revendications 1—11, caractérisé en ce que le mélange réactionnel sortant de la zone de réaction, après avoir été refroidi jusqu'à un certain degré ou non, est conduit dans un solvant immiscible ou pratiquement immiscible à l'eau pour l'isocyanate.

17. Procédé selon revendication 16, caractérisé en ce que un agent finement divisé absorbant l'eau est suspendu dans le solvant.

18. Procédé selon la revendication 14, caractérisé en ce que la température à laquelle le mélange solvant-isocyanate est condensé est choisi de telle manière qu'elle se situe de justesse au-dessus du point de rosée de l'eau contenue dans la mélange réactionnel après que celui-ci a été débarrassé tant du solvant que de l'isocyanate.

19. Procédé selon la revendication 16, caractérisé en ce que la température du solvant à travers lequel est conduit le mélange réactionnel est choisie de telle manière qu'elle se situe de justesse au-dessus du point de rosée de l'eau contenue dans le mélange réactionnel après que celui-ci a été débarrassé tant du solvant que de l'isocyanate.

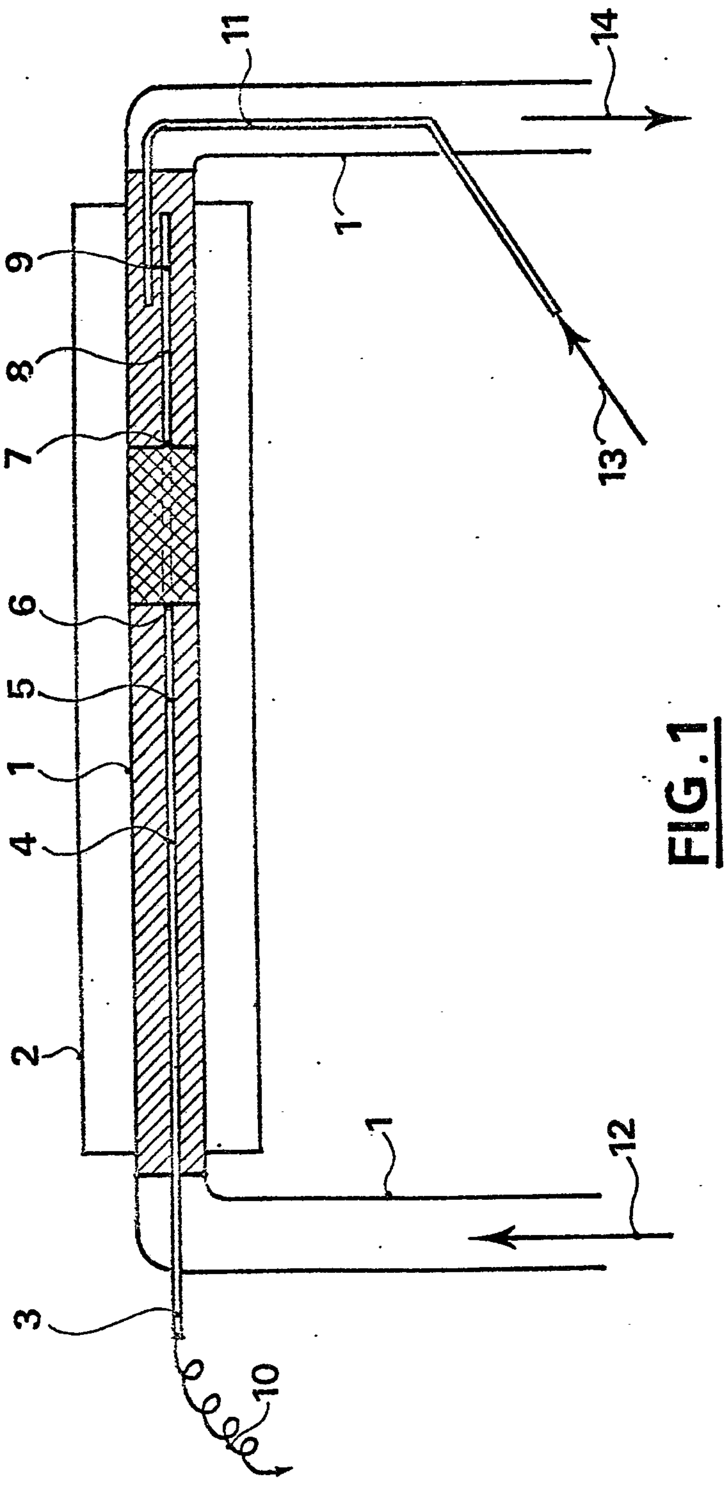


FIG. 1

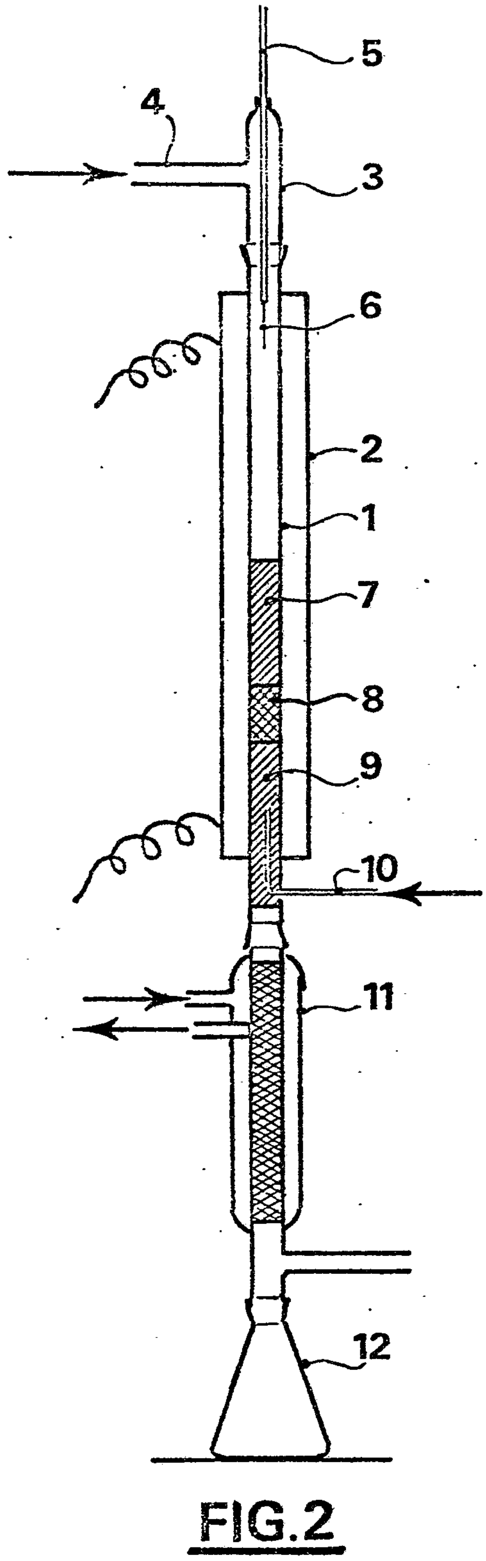


FIG.2